# EUROPÄISCHE PATENTSCHRIFT

(11) **EP 0 499 940 B1**
(45) Veröffentlichungstag und Bekanntmachung des Hinweises auf die Patenterteilung: **03.08.1994**
(21) Anmeldenummer: 92102209.1
(22) Anmeldetag: 10.02.1992
(51) Int. Cl.: H04R 25/02

(54) **Elektromechanischer Wandler für implantierbare Hörgeräte**
Electromechanic transducer for implantable hearing aids
Transducteur électromécanique pour appareils de correction auditive implantables

(30) Priorität: 13.02.1991 DE 4104358
(43) Veröffentlichungstag der Anmeldung: 26.08.1992
(73) Patentinhaber: IMPLEX GmbH Spezialhörgeräte, D-85737 Ismaning (DE)
(72) Erfinder: Leysieffer, Hans, Dr.-Ing., W-8028 Taufkirchen (DE); Hortmann, Günter,, W-7449 Neckartenzlingen (DE); Baumann, Joachim,, W-8000 München 40 (DE)
(74) Vertreter: Schwan, Gerhard, Dipl.-Ing.

(56) Entgegenhaltungen:
- CH-A- 627 604
- DE-C- 3 918 086
- DE-C- 3 940 632

## Beschreibung

Die vorliegende Erfindung betrifft einen elektromechanischen Wandler für implantierbare Hörgeräte für mittlere bis schwere Hörschäden, deren Ursache im wesentlichen im Innenohr lokalisiert ist.

Da eine Hörschädigung heute nach wie vor als ''Behinderung'' angesehen wird, geht der Trend der Entwicklung konventioneller Hörgeräte seit vielen Jahren bis an die Grenze der machbaren Miniaturisierung der Geräte. Die ''Im-Ohr-Geräte'' (IdO) (z.B. DE-OS 28 25 233) haben unter diesem Aspekt einen wesentlichen Fortschritt gebracht und sind heute weiter verbreitet als "Hinter-dem-Ohr-Geräte" (HdO) (z.B. EP 0 341 902). Die Miniaturisierung bringt jedoch folgende Nachteile mit sich:
- deutlich kleinere akustische Verstärkung gegenüber den HdO-Geräten;
- mangelnde Klangqualität, die wesentlich auf nichtlineare Verzerrungen bei hohen Ausgangsschalldruckpegeln und den schlechten Frequenzgang der "Lautsprecher" (Hörer) zurückzuführen ist; diese mindere Qualität ist im wesentlichen durch das Wandlerprinzip (elektromagnetisch) und die extrem kleine Bauform bedingt. Beide Faktoren lassen kaum noch Verbesserungen zu, da hier die physikalischen Grenzen bereits annähernd erreicht sind;
- Rückkopplungspfeifen, das bei nicht dichtem Sitz der Geräte im äußeren Gehörgang und hoher akustischer Verstärkung auftritt;
- trotz Unterbringung im Gehörgang bleiben die meisten Geräte sichtbar; dieser wichtige Aspekt ist ein seit langem bekanntes psychologisches Problem (Stigmatisierung) und spielt eine wesentliche Rolle z.B. im Pubertätsalter oder bei wichtigen gesellschaftlichen Funktionen;
- der aus akustischen Gründen notwendigerweise dichte Sitz der IdO-Geräte im äußeren Gehörgang bedingt den unangenehmen "Verschluß"-Effekt ("Fremdkörper" im Gehörgang).

Ein vollständig implantierbares Hörgerät mit einem optimierten elektromechanischen Wandler zur direkten mechanischen Anregung des Mittel- bzw. Innenohres, das die oben genannten Nachteile nicht aufweist und darüberhinaus wesentliche Verbesserungen mit sich bringt, kommt bei den Hörstörungen in Betracht, deren Ursache im Innenohr oder in höheren Verarbeitungsebenen lokalisiert ist und nicht durch operative Maßnahmen behebbar ist. Der Ansteil einer derartigen Innenohrschädigung (Lärmbelästigung, krankheitsbedingte Schädigung, ototoxische Medikamentierung, Altersschwerhörigkeit etc.) überwiegt dabei weltweit deutlich (ca. 80 %) die Fälle einer operativ rekonstruierbaren Mittelohrschädigung (Schalleitungsstörung).

Aus den genannten Gründen werden seit geraumer Zeit Versuche unternommen, andere Prinzipien der (Innen-)Ohranregung als der Rückwandlung in verstärkten Luftschall zu verwenden. Hier sind z.B. die Möglichkeiten teilimplantierbarer, elektromagnetischer Systeme zu nennen, bei denen eines der Gehörknöchelchen im Mittelohr (Hammer, Amboß und Steigbügel) mit einem sehr kleinen Permanentmagneten mechanisch fest verkoppelt wird; ein (konventionelles) IdO-Hörgerät steuert nun nicht einen Hörer an, sondern eine magnetische Induktionsspule, deren Wechselfeld, das der akustischen Information entspricht, den implantierten Magneten und damit die Ossikelkette zu Schwingungen anregt, die bei geeigneter Systemauslegung in ihrer Amplitude größer sind als im natürlichen Fall und so den Verstärkungseffekt herbeiführen. Hierbei ist die antreibende Spule von außen möglichst nahe an das Trommelfell zu bringen, um den Luftspalt zum implantierten Magneten minimal zu halten. Weitere Ausführungsformen bestehen im Ersatz eines der Ossikel (z.B. Amboß) aus einem biokompatiblen Werkstoff, der einen Permanentmagneten ummantelt. Derartige, teilimplantierbare elektromagnetische Systeme sind in der Literatur z.B. in Heide et al. (Adv. Audiol., vol. 4, pp. 32-43, Karger, Basel 1988) beschrieben und in zahlreichen Patentschriften dokumentiert (US 4 756 312, US 5 015 225, DE 3 617 118 C2, US 4 606 329, UK 2 188 209, EP 0 242 038, US 3 870 832, UK 1 440 724, US 5 015 224).

Daneben sind auch Systeme bekannt, die das Innenohr über eine Körperschall(Knochen-)Leitung anregen (US 2 176 078, teilimplantierbar elektromagnetisch und US 4 150 262, nichtimplantierbar piezoelektrisch).

Die meisten der oben genannten Verfahren und Geräte enthalten teilimplantierbare Wandler zur Anregung der Mittelohr-Ossikel oder direkt des ovalen Fensters.

Ein mögliches Prinzip eines vollständig implantierbaren elektromechanischen Wandlers zur direkten mechanischen Anregung des Steigbügels ist in Publikationen der japanischen Gruppe um Yanigahara und Suzuki et al. beschrieben (Arch Otolaryngol Head Neck, Surg-Vol 113, 1987, pp. 869-872; Hoke, M. (ed), Advances in Audiology, Vol. 4, Karger Basel, 1988).

Bei diesem System besteht der elektromechanisch aktive Teil aus einem piezoelektrischen Keramik-Biegeschwinger, der als Bimorph ausgebildet ist und mit seinem frei schwingenden Ende über ein kleines Koppelelement (z.B. aus Polyethylen) das Köpfchen des Steigbügels direkt antreibt. Die mechanisch notwendige feste Einspannung des anderen Endes dieses Biegeschwingers wird durch ein kompliziertes Justagegestänge aus Titan erreicht, dessen Befestigungselement mit dem Knochen verschraubt ist. Humanversuche in lokaler Anästhesie zeigen, daß mit diesem Wandlerelement bei Wandlerspannungen um 1,0 Volt Auslenkungen des Steigbügels erreichbar sind, die äquivalenten Anregungs-Schallpegeln von 90-100 dB SPL im audiologisch wichtigen Frequenzbereich von ca. 250 - 4000 Hz entsprechen und damit für eine Hörgeräteversorgung durchaus ausreichend sind. Ein wesentliches weiteres Ergebnis dieser Untersuchungen ist die von allen Probanden bestätigte hohe Klangqualität und Verzerrungsfreiheit dieser Anregungsart, die sich auch in den Ergebnisquoten von Sprachverständlichkeitstests bei Probanden mit deutlichen Innenohrhörschäden dokumentiert.

In den oben genannten Publikationen wird deutlich darauf hingewiesen, daß eine wesentliche Problematik bei der technischen Realisierung der beschriebenen Wandlerelemente in der langzeitstabilen biokompatiblen Ummantelung des frei schwingenden piezoelektrischen Keramikelementes liegt, die zudem auch hohe elektrische Isolationswerte im Bereich einiger MOhm aufweisen muß, da derartige Keramikelemente aufgrund ihrer geringen Ruhekapazität eine hohe elektrische Impedanz aufweisen. Eine weitere Schwierigkeit besteht in dem Platzbedarf derartiger Wandlerelemente und der zugehörigen Positionierungsmechanik, der eine sichere und zuverlässige Applikation im Mittelohrbereich unter Berücksichtigung der individuellen Schwankungen der anatomischen Geometrien behindert. Weiterhin muß eine anatomisch normale und damit funktionsfähige Ossikel-Kette unterbrochen werden, um das oben genannte Wandlerelement anwenden zu können; bei Ausfall oder sonstigen technischen oder klinischen Problemen, die die Entfernung des Wandlers indizieren, muß deshalb eine operative Rekonstruktion der Kette versucht werden, um den vorherigen Zustand wiederherzustellen.

Ein ähnliches Verfahren wird von Epley angegeben (US 3 712 962), der ebenfalls piezoelektrische Biegeschwinger mit Ankopplung an den Steigbügel als Wandler verwendet, die als Bimorph oder in Multi-Layer-Technik aufgebaut sind. Hier wird auch auf die Notwendigkeit einer geeigneten Ummantelung hingewiesen (flexible Kunststoffe, Silikon); des weiteren ist eine Ausführungsform von Koppelelementen des freien Biegeschwingerendes zum Stapes beschrieben.

Nunley et al. (US-PS 3 882 285) beschreiben ein Verfahren, bei dem parallel zur anatomisch normalen und intakten Ossikel-Kette über ein piezoelektrisches Element der Steigbügel oder das ovale Fenster direkt in Schwingungen versetzt werden, um den natürlichen Übertragungsweg zu "unterstützen" und zu verstärken. Eine detaillierte Ausführungsform des Wandlers ist nicht angegeben. Hierzu ist zu bemerken, daß bei nicht idealem Phasengang der Auslenkung des Wandlers im Vergleich zur natürlichen Übertragung Interferenzen auftreten können, die erhebliche Einbrüche im gesamten Frequenzbereich mit sich ziehen und damit die Übertragungsqualität und die Verstärkung sehr negativ beeinflussen können.

Ähnliche Angaben sind bei Wingrove (US-PS 3 594 514) dokumentiert, der ebenfalls ein piezoelektrisches Bimorph-Element zur Anregung des Steigbügels oder ovalen Fensters verwendet, das einseitig fest im Mastoid-Knochen verankert sein soll und eine adäuquate biokompatible Kapselung aufweist. Detaillierte Ausführungsformen oder Beschreibungen sind auch hier nicht angegeben.

Weitere verschiedenartige Anregungsformen des Steigbügels oder ovalen Fensters ebenfalls mit piezoelektrischen Biegeschwingern in Bimorph-Bauweise finden sich bei Branch et al. (US-PS 3 764 748); detaillierte Ausführungsformen der Möglichkeiten, wie derartige Wandlerelemente biokompatibel ummantelt und elektrisch isoliert sein können, werden auch nicht genannt.

Die detailliertesten Ausführungsformen adäquater Wandlerverfahren für vollständig implantierbare Hörgeräte finden sich bei Schaefer (US-PS 4 729 366, EP-A1 0 263 254). Hier werden eine Methode der Gehörverbesserung und ein vollständig implantierbares Gerät beschrieben, bei denen nach einer Unterbrechung der Ossikel-Kette (typisch durch Entfernung des Amboß als Bindeglied zwischen Hammer und Steigbügel) zwei elektromechanische Wandler, die ebenfalls vorzugsweise als piezoelektrische Bimorphelemente aufgebaut sind, in die unterbrochene Kette als "aktives Bindeglied" eingesetzt werden, wobei einer der Wandler die Mikrophonfunktion durch mechanische Ankopplung an das Trommelfell bzw. den Hammer erfüllt und das so gewonnene elektrische Signal über ein elektronisches System verstärkt dem Ausgangswandler zugeführt wird. Dieser Stimulationswandler regt ebenfalls wiederum den Steigbügel oder das ovale Fenster an.

In den oben genannten Patentschriften von Schaefer werden denkbare Ausführungsformen dieser Wandler dahingehend beschrieben, daß vorzugsweise piezoelektrische Biegeschwinger einseitig in nicht näher beschriebenen Gehäusen eingespannt werden und die Ankopplung des freien, schwingfähigen Endes dieser Wandler an die Ossikel vorzugsweise über einen dünnen Draht aus rostfreiem Stahl geschieht, wobei bei dem Ausgangswandler dieser Draht z.B. um das Steigbügelköpfchen geschlungen wird. Die seitliche Führung dieses Drahtes durch die Wandung des Gehäuses, das das aktive Wandlerelement enthält, geschieht über eine Führungshülse, die in die Gehäusewand eingelassen ist. Eine präzise Erläuterung dieser Ausführungsform ist jedoch nicht angegeben. Die aktiven Wandlerelemente können piezoelektrisch (piezoaktive Polymere wie PVDF oder Piezokeramiken, vorzugsweise mit Bimorphstruktur) oder elektromagnetisch sein.

Der oben angeführte Stand der Technik vollständig implantierbarer elektromechanischer Wandler für Hörgeräte kann wie folgt zusammengefaßt werden:
- Bei annähernd allen Publikationen und Patentschriften wird die Verwendung piezoelektrisch aktiver Wandlerelemente vorgeschlagen, die ausschließlich als Bimorph-Struktur oder in Multilayer-Technik realisiert sind. Das Prinzip des Bimorph besagt, daß zwei Streifen piezoelektrisch aktiven Materials so mechanisch zusammengefügt sind, daß bei gleichzeitiger elektrischer Anregung der beiden einseitig mechanisch fest eingespannten Elemente sich eines aufgrund des piezoelektrischen Quereffekts verkürzt und sich das andere entsprechend verlängert, wodurch eine deutlich größere Durchbiegung und damit Auslenkung des freien Endes dieses Verbundelements erreicht wird als mit nur einem Wandlerstreifen. Bei Zusammenfügung mehrerer als zweier dieser Streifen (Multilayer-Technik) kann zusätzlich die erzeugbare Kraft erhöht werden. Dieses Prinzip ist dann weitgehend optimal genutzt, wenn die Geometrie der Verbundelemente als rechteckförmiger Streifen ausgelegt wird, wobei mit steigender Länge und abnehmender Dicke der Verbundelemente die erzielbare Auslenkung bei gegebener Wandlerspannung zunimmt.
- Andere physikalische Wandlerprinzipien werden zwar erwähnt (insbesondere elektromagnetisch), doch ist klargelegt, daß nur mit dem piezoelektrischen Effekt technisch einfache Wandlerelemente realisierbar sind.
- Die erreichbare Klangqualität bei Anregung der Ossikelkette und insbesondere des Steigbügels direkt mit den oben genannten Bimorphelementen ist offenbar sehr hoch.
- In einigen Patentschriften und Publikationen finden sich zwar Hinweise auf die notwendige biokompatible Ummantelung und hochwertige elektrische Isolation dieser piezoelektrischen Wandlerelemente; detaillierte Ausführungsformen derartiger Wandlerkonstruktionen sind jedoch, wenn überhaupt, nur angedeutet.
- Die langjährigen Erfahrungen mit implantierbaren, aktiven Human-Implantaten, wie Herzschrittmachern und Cochlea Implants, zeigen, daß derartige Systeme hermetisch dicht sein müssen, um dem Anspruch der klinischen Sicherheit und einer langen Lebensdauer zu entsprechen. Der Begriff der hermetischen Dichtheit wird jedoch in keiner der vorstehend genannten Patentschriften erwähnt; insbesondere wird kein implantierbares Hörgerät mit einer für hermetische Dichtheit sorgenden Wandlerkonstruktion beschrieben.
- Gleichfalls wird nicht beschrieben, wie eine derartige Wandlerkonstruktion ausgelegt sein muß, damit der Wandler auch tatsächlich implantierbar ist, das heißt, so gestaltetwird, daß der Operateur weitgehend freie Sicht aufdas Operationsfeld behält (insbesondere aufdie Ossikelkette und das ovale und runde Fenster); weiterhin muß der Wandler so konstruiert sein, daß dessen Applikation im Mittelohrbereich unter den gegebenen anatomischen Verhältnissen möglich ist und keine weitgehenden operativen Ausräumungen vorgenommen werden müssen, die aufwendig sind und das hohe Risiko traumatischer Reaktionen und späterer Infektionen mit sich bringen. Dieser Aspekt widerspricht der Anwendung piezoelektrischer Bimorph-Wandlerelemente, da diese -wie erwähnt- technisch optimal längsgestreckt sein müssen und eine feste Einspannung eines Endes erfordern (minimale sinvolle Länge ca.7 mm; dieses Maß überschreitet bereits anatomisch durchschnittliche Geometrien im Mittelohr). Derartige Konstruktionsmerkmale sind ebenfalls in keiner Patentschrift angegeben.
- Direkte Wandlermaterialangaben für piezoelektrische Elemente beziehen sich lediglich auf Begriffe wie "piezoelektrisch aktive Keramik" oder PVDF (Polyvinylidenfluorid).

Lediglich in zwei der genannten Patentschriften sind Lösungsansätze zur vollständigen Kapselung implantierbarer Hörgeräte-Wandler angegeben (US-4 988 333 und DE 3 918 086) Hier werden aktive Wandlerelemente in geschlossenen Gehäusen verwendet, deren elektrisch angeregte mechanische Schwingungen über Schlauchleitungen ausgekoppelt werden; die Oszillationen werden in diesen Schlauchleitungen in flüssigen oder gasförmigen Medien weitergeleitet und entweder an das Köpfchen des Steigbügels geführt oder direkt über eine Flüssigkeitskopplung durch das ovale oder das runde Fenster in die Perilymphe der cochleären Basalwindung. Ein wesentlicher Vorteil dieser speziell hydromechanischen Kopplung (DE 3 918 086) besteht darin, daß auf der Basis des hydraulischen Prinzips am Ende des Koppelelementes größere Auslenkungen im Vergleich zur antreibenden Wandlermembran und damit hohe äquivalente Ausgangsschalldruckpegel erreichbar sind. Problematisch sind hier im Hinblick auf die technische Realisation hygienische Fragen der keimfreien Flüssigkeitsfüllung und der langzeitstabilen Ankopplung an das Mittel- bzw.Innenohr.

Die vorliegende Erfindung setzt sich zur Aufgabe, einen elektromechanischen Wandler zur direkten mechanischen Anregung des Mittel/Innenohres für vollständig implantierbare Hörgeräte zu schaffen, der den bekannten Stand der Technik dahingehend verbessert, daß einerseits die oben genannten Nachteile der dokumentierten Wandlermethodiken vermieden werden und andererseits die Betriebseigenschaften des Wandlers eine hohe Klangqualität und ausreichende Schalldruckpegel für eine adäquate Hörgeräteversorgung ermöglichen.

Ein elektromechanischer Wandler für implantierbare Hörgeräte zur direkten mechanischen Anregung des Mittel- oder Innenohres ist erfindungsgemäß gekennzeichnet durch ein hermetisch dichtes und biokompatibles Gehäuse, bei dem eine Gehäusewand als schwingfähige Membran ausgeführt ist, die mit einer auf der Innenseite aufgebrachten piezoelektrischen Keramikscheibe ein elektromechanisch aktives Heteromorph-Verbundelement darstellt, und deren mechanische Schwingungen über einen auf der Außenseite der Membran fest fixierten, mechanisch steifen Bügel zusammen mit einem mechanisch steifen Koppelelement auf ein Mittelohr-Ossikel oder direkt auf das Innenohr übertragen werden.

Erfindungsgemäß wird also die vorstehend genannte Aufgabe dadurch gelöst, daß ein piezoelektrisch aktives Element in einem vollständig gekapselten, hermetisch dichten und biokompatiblen Gehäuse so untergebracht wird, daß dieses Piezoelement in unmittelbarer mechanischer Verbindung mit einer aus dünnem Material ausgeführten Wand des kapselnden Gehäuses steht und so diese Gehäusewand als aktive schwingende Membran agiert, deren Schwingungen über ein außen an dieser Membran fest angebrachtes, mechanisch steifes Koppelelement an das Mittel- bzw. Innenohr weitergeleitet werden.

Dabei ist Kernpunkt der Erfindung das Prinzip eines hermetisch dichten, piezoelektrischen Heteromorph-Wandlers, der elektronisch verstärkte (Audio-)Signale in mechanische Schwingungen umwandelt und diese unmittelbar ohne Rückwandlung in akustische Signale an das Mittel- bzw. Innenohr weiterleitet.

Bei der Wandlerauslegung nach der Erfindung wird wie bei einem Bimorphelement der piezoelektrische Quereffekt genutzt, nur besteht der Partner des Verbundes hier nicht aus einem zweiten piezoelektrisch aktiven Element, sondern aus einer passiven, metallischen Membran ähnlicher Geometrie wie das Piezoelement. Dearartige Verbundelemente werden piezoelektrische Heteromorph-Wandler genannt und sind heute z.B. als akustische Signalgeber in Personal-Computern weit verbreitet. Solche Wandlerelemente sind meist kreisförmige, piezoelektrische Keramikscheiben, die beidseitig mit sehr dünnen, elektrisch leitfähigen Materialien beschichtet sind, welche als Elektrodenflächen dienen. Diese Piezoscheiben sind mit elektrisch leitfähigen Klebstoffen einseitig mit metallischen Membranen (meist Messing) etwa gleicher Dicke, aber größeren Durchmessern mechanisch fest verbunden. Wird ein elektrisches Feld über die beiden Elektrodenflächen (ein Anschluß ist in diesem Applikationsfall die metallische Membran) an die piezoelektrische Scheibe gelegt, verändert die Scheibe aufgrund des transversalen Piezoeffektes (Quereffekt) ihre Geometrie vorzugsweise in radialer Richtung. Da eine Ausdehnung bzw. radiale Verkürzung jedoch durch den mechanisch festen Verbund mit der passiven, metallischen Membran verhindert wird, ergibt sich eine Durchbiegung des Verbundelements, die bei entsprechender Randlagerung der Scheibe in der Mitte maximal ist.

Dieses Prinzip des piezoelektrischen Heteromorph-Wandlers kann vorteilhaft zur Lösung der vorliegenden Problematik eingesetzt werden.

Weitere Ausgestaltungen der Erfindung ergeben sich aus den Unteransprüchen.

Bevorzugte Ausführungsbeispiele der Erfindung sind nachstehend anhand der Zeichnungen näher erläutert. Es zeigen:
- Fig. 1: einen vergrößerten schematischen Schnitt durch einen erfindungsgemäß ausgebildeten elektromechanischen Wandler,
- Fig. 2: die Verwendung des elektromechanischen Wandlers zur direkten Innenohranregung durch das ovale Fenster,
- Fig. 3: eine Anordnung ähnlich Fig. 2, die eine abgewandelte Art der Fixation des Wandlergehäuses an der Implantationsstelle erkennen läßt,
- Fig. 4: in größerem Maßstab die Ausbildung eines Koppelelements zum Steigbügel-Köpfchen,
- Fig. 5: die Verwendung eines Koppelelements zur direkten Innenohrankopplung über das ovale Fenster,
- Fig. 6: die Ankopplung an den natürlichen Amboß bei intaktem Amboß-Steigbügelgelenk und
- Fig. 7: eine weitere Art der Positionierung des Wandlergehäuses.

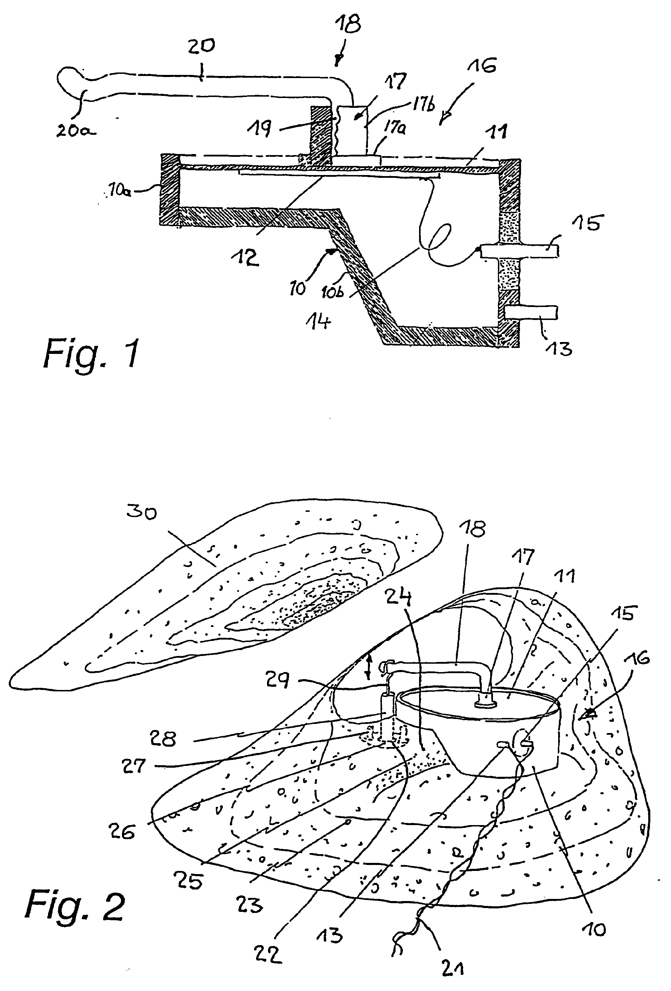

Bei dem in Fig.1 veranschaulichten Ausführungsbeispiel ist die Oberseite eines metallischen, allseitig hermetisch dicht geschlossenen, biokompatiblen Gehäuses 10 als vorzugweise kreisförmige, dünne, schwingungsfähige Membran 11 ausgeführt. Innerhalb des Gehäuses 10 ist die Membranwand 11 unter Verwendung eines elektrisch leitfähigen Klebstoffes mit einer gleichfalls vorzugsweise kreisförmigen piezoelektrisch aktiven Keramikscheibe 12 mechanisch fest verbunden. Der Durchmesser der Keramikscheibe 12 ist kleiner als der Durchmesser der Gehäusemembran 11. Die von der einen Gehäusewand 11 und der Keramikscheibe 12 gebildete Anordnung stellt ein elektromechanisch aktives Heteromorph-Verbundelement dar. Die Oberflächen der Piezoscheibe 12 sind, im Vergleich zur Scheibendicke dünn, mit elektrisch leitfähigem Material als Elektroden beschichtet. Ein elektrischer Anschluß (Masseanschluß) 13 ist an dem metallischen und damit elektrisch leitfähigen Gehäuse 10 direkt angebracht. Die Gegenelektrodenfläche auf der Unterseite der Piezoscheibe 12 ist mit einem dünnen, flexiblen Draht 14 kontaktiert (z.B. mit Bond-Techniken). Der Draht 14 ist an eine hermetisch dichte und elektrisch isolierte Gehäusedurchführung 15 angeschlossen, und er bildet so den zweiten elektrischen Anschluß des insgesamt mit 16 bezeichneten Wandlers. Das Wandlergehäuse 10 besteht, einschließlich der Gehäusemembran 11, aus einem biokompatiblen Werkstoff, vorzugsweise Titan, Niob, Tantal oder deren Legierungen, oder aus einem anderen biokompatiblen Metall. Es kann mindestens zum Teil aber auch aus einem körperverträglichen keramischen Werkstoff, zum Beispiel Al₂O₃, gefertigt sein. Das Gehäuse 10 hat zweckmäßig einen Durchmesser im Bereich von 6 bis 13 mm, vorzugsweise etwa 9 mm. Die piezoelektrische Keramikscheibe 12 kann unter anderem aus Blei-Zirkonat-Titanat (PZT) bestehen. Ihre Dicke ist zweckmäßig etwa gleich groß wie die Dicke der Membran 11. Die Dicke der Membran 11 und der Keramikscheibe 12 liegen vorteilhaft jeweils im Bereich von 0,05 bis 0,15 mm. Bei kreisförmiger Ausbildung des Heteromorph-Verbundelements 11/12 sollte der Radius der Gehäusemembran 11 um den Faktor 1,2 bis 2,0 größer sein als der Radius der piezoelektrischen Keramikscheibe 12. Als besonders günstig hat sich ein Faktor von etwa 1,4 erwiesen.

Wird an die beiden elektrischen Anschlüsse 13, 15 von einem (nicht veranschaulichten) Elektronikmodul über eine Zuleitung 21 eine elektrische Spannung gelegt, ergibt sich eine radiale Geometrieänderung der Piezoscheibe 12, die zu einer Durchbiegung des Hetero-Verbundes Piezokeramik-Metallmembran und damit zu einer Auslenkung der Metallmembran 11 führt. Wegen der randharten Einspannung (Schnelle gleich Null) der Membran ist die Membranauslenkung in der Mitte maximal.

Auf der außenliegenden Seite der Gehäusemembran 11 ist mittig (z.B. durch Schweißen oder mit biokompatiblen Klebstoffen) ein kreisförmiger Flansch 17 mechanisch fest aufgebracht. Der Durchmesser des Flanschs 17 ist wesentlich kleiner als der Membrandurchmesser. Mit dem Flansch 17 ist der kurze Schenkel 19 eines im wesentlichen L-förmigen, dünnen Bügels 18 mechanisch fest verbunden. Unmittelbar nach Austritt aus dem Flansch 17 biegt der Bügel 18 im rechten Winkel ab, so daß sein langer Schenkel 20 mindestens annähernd parallel zur Gehäuseoberfläche verläuft. Der lange Schenkel 20 des Bügels 18 ist geringfügig länger als der Radius der kreisförmigen Gehäuseoberfläche, so daß er über die Gehäuseaußenkante geringfügig, vorzugsweise etwa 2 mm, übersteht. Der Bügel 18 ist konstruktiv so gestaltet und aus solchem Material beschaffen, daß eine möglichst hohe mechanische Steifigkeit bei möglichst geringer Masse erreicht wird. Insbesondere kann der Bügel 18 aus metallischen Voll- oder Hohldraht oder aus einem kohlefaserverstärkten Verbundwerkstoff mit hohem Elastizitätsmodul gefertigt ein. Der Durchmesser des Bügels 18 liegt zweckmäßig im Bereich von 0,25 bis 1,0 mm; er beträgt vorzugsweise etwa 0,5 mm.

Durch die mechanisch feste Verkopplung des Flanschs 17 mit der Gehäusemembran 11 führt der Bügel 18 bei Durchbiegung der Gehäusemembran 11 eine zur Gehäuseoberfläche senkrechte Bewegung aus. Die Durchbiegung der Gehäusemembran 11 und damit die senkrechte Auslenkung des Bügels 18 folgen aufgrund der elektromechanischen Wandlung des piezoelektrischen Elements 12 dem zeitlichen Verlauf der an die Anschlußklemmen 13, 15 des Wandlers 16 gelegten Spannung. Infolgedessen führen die von einem (nicht dargestellten) Mikrofon aufgenommenen und elektrisch gewandelten Audiosignale nach elektronischer Verstärkung in dem Elektronikmodul unmittelbar zu mechanischen Auslenkungen, welche der akustischen Information entsprechen. Diese senkrechten Auslenkungen des Bügels 18 werden durch mechanische Koppelelemente, die an dem leicht gebogenen äußeren Ende des langen Schenkels 20 des Bügels 18 fest fixiert werden können, an die Ossikelkette des Mittelohrs beziehungsweise an den Steigbügel oder das ovale beziehungsweise runde Fenster oder auch ein artifizielles Fenster weitergeleitet. Sie bewirken so bei entsprechender Auslegung des vorverarbeitenden elektronischen Systems den audiologischen Verstärkungseffekt.

Die Dimensionierung des Verbundelements Piezoscheibe 12/Metallmembran 11 mit mechanisch fest fixiertem Bügel 18 ist vorzugsweise so ausgeführt, daß die dynamischen Massenbeläge und Steifigkeiten eine Hauptresonanzfrequenz (Mode 0/0) im Bereich von 6 bis 12 kHz, vorzugsweise etwa 10 kHz, ergeben, das heißt, eine erste mechanische Resonanzfrequenz, die an der oberen Grenze des angestrebten Übertragungsbereiches (beispielsweise ca. 100 Hz bis 12 kHz) liegt. Das bedeutet, daß das System hochabgestimmt ist. Dies hat einerseits den wesentlichen Vorteil, daß im tiefen bis mittleren Frequenzbereich ein ebener Frequenzgang der Wandlerauslenkung und damit ideale Übertragungseigenschaften erreicht werden. Andererseits ergibt sich durch die untere Flanke der Resonanzüberhöhung, die noch im Übertragungsbereich liegt, ein Anstieg der Wandlerauslenkung bei unveränderter Wandlerspannung. Damit stehen hohe Anregungspegel gerade in dem Frequenzbereich zur Verfügung, in dem erfahrungsgemäß die meisten Innenohrschwerhörigkeiten beginnen und/oder am ausgeprägtesten sind. Zusätzlich wird in diesem oberen Frequenzbereich die subjektive Lautstärkeempfindung höher, weil der Frequenzgang der natürlichen Steigbügelauslenkung ab ca. 1 kHz mit zunehmender Frequenz stark abfällt (zum Beispiel Gyo, K. und Goode, R. L.: "Measurement of Stapes Vibration Driven by the Ceramic Vibrator of a Middle Ear Implant - Human Temporal Bone Experiments", Adv. Audiol., vol. 4, pp. 107-116, Karger, Basel 1988).

Aus der schematischen Schnittdarstellung des Wandlers 16 in Fig.1 ist ersichtlich, daß das zweckmäßig mit einem Edelgas, zum Beispiel Argon, gefüllte Gehäuse 10 nur im Bereich der Gehäusemembran 11 in Form einer Kreisscheibe ausgeführt ist. Der der Membran abgewandte Teil 11 des Gehäuses 10 ist nach unten und entgegengesetzt zur Austrittsrichtung des Bügels 18 verdickt, sodaß eine Abstufung vorliegt und die annähernde Form eines "Schuhs" entsteht. Aus Fig.1 ist ersichtlich, daß in diesem verdickten Gehäusebereich Raum für die hermetisch dichte Kontaktdurchführung für den aktiven elektrischen Anschluß 15 des Piezoelements 12 vorhanden ist.

Diese stufige Ausführung des unteren Teils des Wandlergehäuses 10 hat noch einen zweiten Grund, der aus Fig. 2 ersichtlich ist, welche die Position des Wandlers 16 und eine mögliche Ankopplungsform, hier direkt zum Innenohr durch das ovale Fenster 22, darstellt. Aus dieser Darstellung geht hervor, daß das Wandlergehäuse 10 nicht im Mittelohr (Paukenhöhle) untergebracht ist, sondern in einem Bereich, der vom Mastoid her zugänglich ist. Dabei wird der Wandler nach Eröffnung des Mastoids in einer artifiziellen Mastoidhöhle 23 von hinten so an das Antrum geführt, daß die Längsachse des abgewinkelten Bügelteils in Richtung Mittelohr weist. Der kreisförmige Membranbereich ruht dabei aufeinem knöchernen Wall 24 über dem Facialis-Kanal 25, beziehungsweise dem horizontalen Bogengang, während der nach unten verdickte Teil des Wandlergehäuses 10 auf dem Boden der Mastoidhöhle 23 aufsitzt. Die Wandlerform ist so optimiert, daß bei dieser Position des Wandlergehäuses 10 das leicht gekrümmte Ende des Bügels 18 möglichst dicht über oder neben dem Stapesköpfchen ruht, so daß verschiedenartige mechanische Elemente zur Ankopplung an die Ossikelkette beziehungsweise direkt an das Innenohr appliziert werden können. Die Ankopplung des schwingenden Wandlerteils an das Mittelohr bzw. an das Innenohr ist unter freier Sicht des Operateurs, z.B. Sicht durch den Gehörgang, möglich.

In Fig. 2 sind aus Gründen der Übersichtlichkeit die Gehörknöchelchen bis auf die Steigbügelfußplatte 26 und die Andeutung der Steigbügelschenkel 27 weggelassen. Beispielhaft ist die direkte Innenohranregung durch das ovale Fenster 22 dargestellt, wobei als Koppelelement eine konventionelle Stapesprothese 28 verwendet werden kann, die nach Eröffnung der Steigbügelfußplatte 26 eingeführt wird und die mit ihrer metallischen Befestigungsöse 29 nun nicht am natürlichen Amboß fixiert wird, sondern an dem gekrümmten Ende des Wandlerbügels 18.

Ein großer Vorteil dieser Wandlerausführung liegt darin, daß die Applikation der Koppelelemente und deren Ankopplung an das Mittelohr-/Innenohr unter voller und direkter Sicht des Operateurs durch den in Fig.2 bei 30 angedeuteten Gehörgang geschehen kann. Ein weiterer Vorteil ist das weitgehende Freibleiben des Mittelohrraums (Paukenhöhle) von großvolumigen Fremdkörpern.

Die Fixation des Wandlergehäuses 10 kann nach optimaler Positionierung mit einem biokompatiblen verklebenden Werkstoff, insbesondere Knochenzement, erfolgen. Eine vorteilhafte Ausführungsform ist auch eine Beschichtung der Wandlergehäuse-Unterseite einschließlich deren Stufe mit bioaktiven Keramiken (z.B. Hydroxylapathit), die ein festes Einwachsen und damit die langzeitstabile Position des Wandlers 16 ermöglichen.

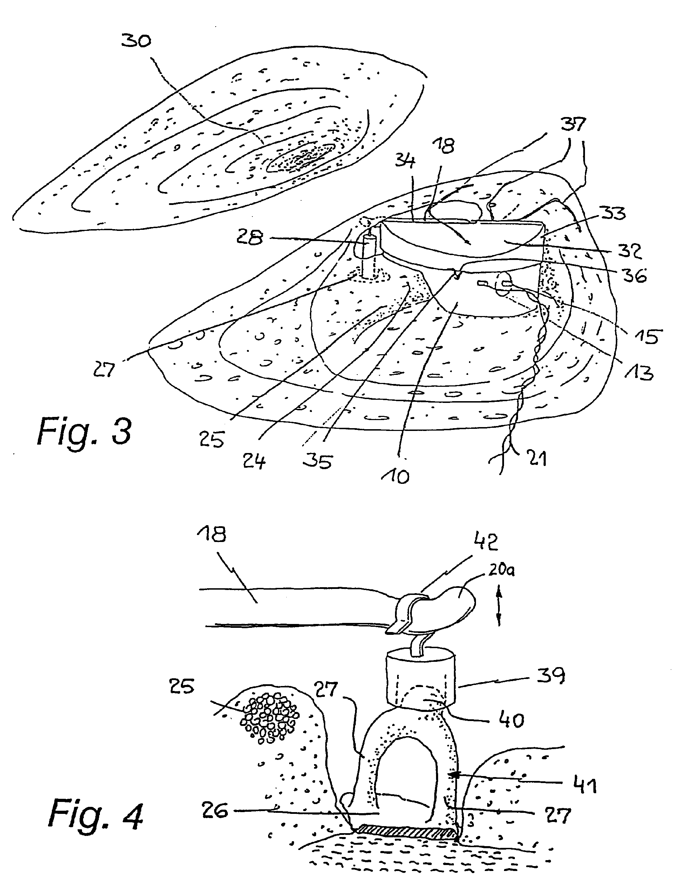

Eine andere Art der Fixation des Wandlers 16 vor Ort ist in Fig.3 dargestellt. Dort ist die Oberfläche des Wandlergehäuses 10 (Membran 11 mit angesetztem Bügel 18) durch einen mit zwei Dachschrägen 32 versehenen Bügel 33 abgedeckt, der am Rand auf der Wandleroberkante aufsitzt. Der Bügel 18 läuft dabei in einem U-förmigen Schlitz 34 in diesem Deckel 33, und er ist somit weitgehend mechanisch geschützt. Dervon oben aufsetzbare Deckel 33 ist, zum Beispiel durch Zusammenwirken einer Nut 35 im Gehäuserand mit einer in die Nut 35 eingreifenden Nase 36 am Deckel 33, verdrehsicher auf dem Wandlergehäuse 10 positioniert. Der Deckel 33 trägt auf der Oberseite zwei oder mehr federnde, vorzugsweise drahtförmige, steife Klammern 37, die zweckmäßig aus Platin, Titan, Niob oder deren Legierungen, oder aus Edelstahl bestehen. In Fig.3 sind drei solche Klammern 37 dargestellt. Die Klammern 37 sind so gestaltet, daß sie das Einführen des Wandlers 16 vom Mastoid aus in der soeben genannten Weise gestatten und nach endgültiger Positionierung so aufgespreizt werden können, daß ein Abstützen an der darüberliegenden Knochenwand an zwei oder mehr Punkten derart stattfindet, daß das Wandlergehäuse 10 nach unten und schräg in Richtung Mittelohr gedrückt wird und somit an dem knöchernen Wall 24 fest anliegt. Zusätzlich kann eine Stabilisierung mit den vorstehend angegebenen Methoden (Knochenzemente, bioaktive Keramikbeschichtungen) erfolgen.

Ein weiterer Vorteil des Gehäusedeckels 33 ist die abschirmende und damit dämpfende Wirkung auf Schallwellen, die in gewissem Umfang von der schwingenden Gehäusemembran 11 abgestrahlt werden und die bei hohen Verstärkungsgraden eventuell zu Rückkopplungsproblemen mit dem schallaufnehmenden Mikrofon führen können, wenn dieses Mikrofon oder eine entsprechende akustische Zuleitung zum Mikrofon in der Nähe des Wandlers 16 positioniert wird.

In den Fign.4 bis 6 sind mögliche Ankopplungsformen des überstehenden Endes des vertikal schwingenden Membranbügels 18 an die Ossikelkette beziehungsweise direkt an das Innenohr dargestellt. Hierbei ist ausdrücklich darauf hinzuweisen, daß für die Applikation des beschriebenen Wandlers die Gehörknöchelchenkette nicht zwingend unterbrochen werden muß. Zur Vermeidung von Interferenzen, die durch die zusätzliche natürliche Schwingung der Ossikelkette durch von außen auf das Trommelfell auftreffende Schallwellen entstehen können, ist eine Immobilisierung der Kette, zum Beispiel durch mechanische Fixation des Hammerkopfes, völlig ausreichend. Es muß nur dafür gesorgt werden, daß die nachfolgenden Ossikel (Amboß und Steigbügel) mobil bleiben, wenn eine Ankopplung an diese Ossikel erfolgen soll.

In Fig.4 ist eine Ausführungsform eines Koppelelements zum Steigbügel-Köpfchen nach natürlicher oder artifizieller Unterbrechung der Ossikelkette (z. B. Fehlen oder Entfernung des Amboß) dargestellt. Eine kleine Hülse 39 aus biokompatiblem Material (beispielsweise Polyethylen, Polytetrafluorethylen oder Hydroxylapathit) ist auf das Köpfchen 40 des insgesamt mit 41 bezeichneten Steigbügels aufgesetzt und mit dem anderen Ende über ein dünnes, von konventionellen Stapesprothesen her bekanntes Metallbändchen 42 (beispielsweise aus Platin, Platinlegierungen oder rostfreiem Stahl) durch weitgehendes Umschlingen mit dem leicht gekrümmten Ende des Wandlerbügels 18 verbunden. Die einfache und sichere Positionierung dieses Koppelelements ist durch die zunächst lose Umschlingung möglich. Die feste mechanische Verbindung zum Bügel 18 kann dann durch Zudrücken des Metallbändchens 42 oder durch Applikation eines Klebstofftropfens (z.B. Medical Grade Silicone) erfolgen.

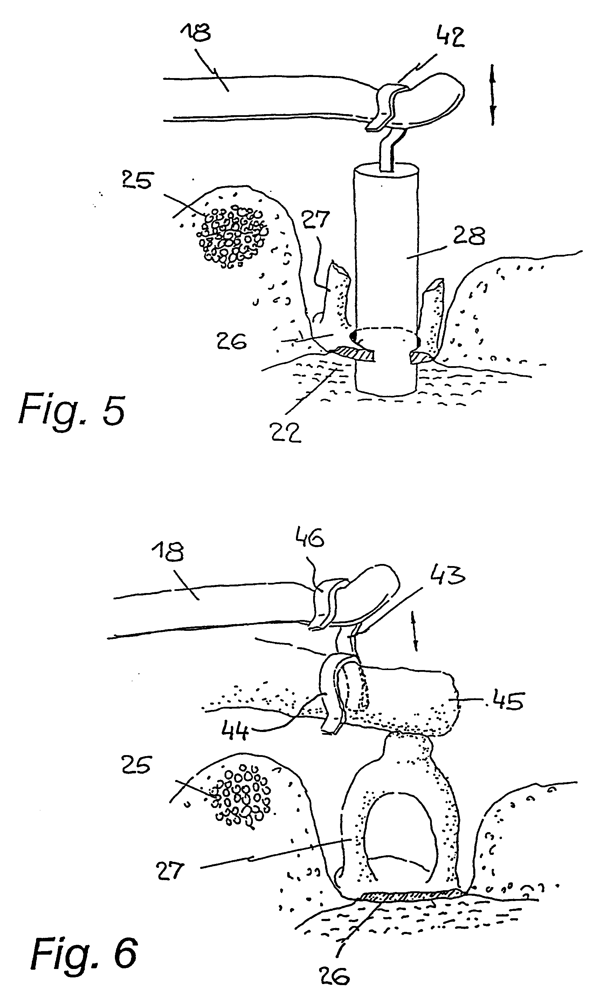

Fig.5 zeigt eine geeignete Methode der direkten Innenohrankopplung über das ovale Fenster 22 unter Verwendung einer standardmäßig verfügbaren Stapesprothese 28. Diese Ankopplungsart erlaubt die weitverbreitete Methodik der Stapedektomie mit Durchbohren oder Entfernen der Fußplatte 26 und Einsetzen einer Prothese 28, die nun nicht an den natürlichen Amboß gehängt wird, sondern wiederum an das Ende des Wandlerbügels 18. Die Positionierung und Fixation des Koppelelements 28 erfolgt wie vorstehend beschrieben.

Fig.6 stellt eine Ankopplungsmethodik an den natürlichen Amboß bei intaktem Amboß-Steigbügelgelenk nach Immobilisieren des Ossikelketten-Eingangs (Hammerkopf) dar. Ein nach Art eines Doppelhakens geformtes Koppelglied 43 umschließt mit einer Klammer 44 (Bändchen zweckmäßg aus Platin, Platinlegierung oder rostfreiem Stahl) den langen Amboßfortsatz 45 wie die Öse einer Stapesprothese. Eine zweite Öse 46 des Koppelgliedes 43 ist wie vorstehend beschrieben an den Wandlerbügel 18 gekoppelt. So wird annähernd die gleiche Schwingungsrichtung des Steigbügels 41 erreicht wie bei natürlicher Anregung.

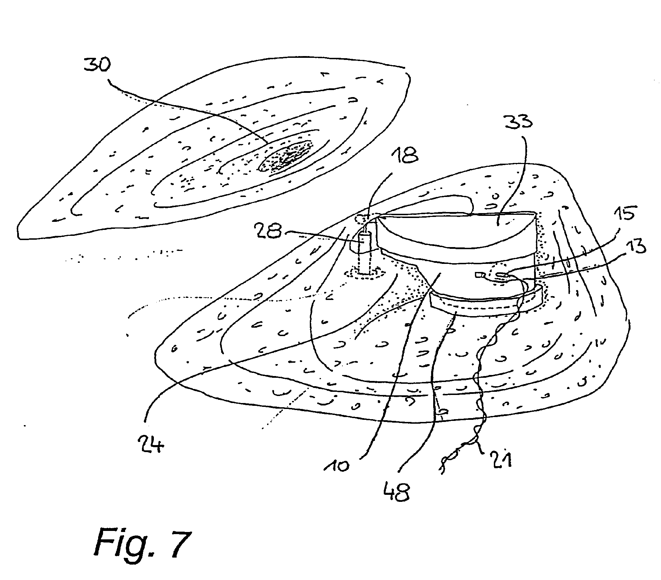

Bei der in Fig. 7 veranschaulichten Ausführungsform ist das Wandlergehäuse 10 in einer beispielsweise pfannenförmig ausgebildeten Haltevorrichtung 48 mechanisch fest und auswechselbar fixiert. Die Haltevorrichtung 48 besteht aus einem bioaktiven Material, das ein langzeitstabiles, mechanisch festes Verwachsen im Antrum gewährleistet.

Zusammenfassend kann das erläuterte Wandlerprinzip so beschrieben werden, daß bei einem hermetisch dichten und biokompatiblen metallischen Gehäuse eine Wandung als Membran ausgebildet ist, die Teil eines piezoelektrischen Heteromorph-Verbundelements ist. Die mechanischen Schwingungen dieser Gehäusemembran werden über ein vorzugsweise am Membranmittelpunkt fest fixiertes, mechanisch steifes Element ausgekoppelt. Dieses Element ist als Bügel so gestaltet, daß es bei Positionierung des Wandlergehäuses vom Mastoid aus an die Paukenhöhle angrenzend, in Form und örtlicher Lage einen artifiziellen Amboß darstellt, an dem geeignete Koppelelemente fixiert werden können, welche die Schwingungen dieses künstlichen Ossikels mechanisch auf das Innenohr übertragen. Dabei ist berücksichtigt, daß einerseits unterschiedliche Koppelelemente verwendet werden können, die der jeweiligen, individuellen pathologischen Situation entsprechen, und daß andererseits die Applikation des gesamten Wandlersystems unter freier Sicht des Operateurs ohne weitreichende, raumschaffende Eingriffe in die anatomischen Gegebenheiten des Mittelohres vorgenommen werden kann.

Der vorstehend beschriebene piezoelektrische Heteromorph-Wandler wurde in Form von Lebormodellen realisiert und in intensiven klinischen Vortests am Menschen erprobt. Dabei wurde bei freiwilligen Patienten, die sich aufgrund geringfügiger Mitelohrdefekte einer rekonstruierenden Mittelohroperation in lokaler Anästhesie unterziehen mußten, die Schwingungen der Wandlermembran von außen durch den Gehörgang über ein verlängertes mechanisches Koppelelement an die Ossikel des Mittelohres angekoppelt, und zwar an den Amboß bei intaktem Amboß-Steigbügelgelenk oder an den Steigbügel beziehungsweise die Steigbügelfußplatte bei defektem oder fehlendem Amboß. Die theoretisch vorherberechneten Betriebsparameter der Wandler konnten bei diesen Vortests in der Praxis bezüglich erreichbarem Stimulationspegel bei geringen elektrischen Wandlerspannungen und erzielbarer Klangqualität voll bestätigt werden.

## Patentansprüche

1. Elektromechanischer Wandler für implantierbare Hörgeräte zur direkten mechanischen Anregung des Mittel- oder Innenohres, gekennzeichnet durch ein hermetisch dichtes und biokompatibles Gehäuse (10), bei dem eine Gehäusewand als schwingfähige Membran (11) ausgeführt ist, die mit einer auf der Innenseite aufgebrachten piezoelektrischen Keramikscheibe (12) ein elektromechanisch aktives Heteromorph-Verbundelement darstellt, und deren mechanische Schwingungen über einen auf der Außenseite der Membran fest fixierten, mechanisch steifen Bügel (18) zusammen mit einem mechanisch steifen Koppelelement (28, 29, 39, 40, 42, 43) auf ein Mittelohr-Ossikel oder direkt auf das Innenohr übertragen werden.

2. Elektromechanischer Wandler nach Anspruch 1, dadurch gekennzeichnet, daß der mechanisch steife Bügel (18) mindestens näherungsweise am Membranmittelpunkt fixiert ist.

3. Elektromechanischer Wandler nach Anspruch 1 oder 2, dadurch gekennzeichnet, daß die Oberseite des Wandlergehäuses (10) die Membran (11) ist und daß die Membran (11) kreisförmig ist.

4. Elektromechanischer Wandler nach einem der vorhergehenden Ansprüche, dadurch gekennzeichnet, daß die Unterseite des Wandlergehäuses (10) derart stufig ausgeführt ist, daß das Wandlergehäuse in einer artifiziell geschaffenen Mastoidhöhle an die Paukenhöhle angrenzend im Antrum so positionierbar ist, daß der Membranbereich nahe den Mittelohr-Ossikeln liegt, die Stufe der Gehäuseunterseite auf dem knöchernen Boden der Mastoidhöhle aufsitzt und die Ankopplung des schwingenden Wandlerteils an das Mittelohr oder das Innenohr unter freier Sicht des Operateurs möglich ist.

5. Elektromechanischer Wandler nach einem der vorhergehenden Ansprüche, dadurch gekennzeichnet, daß das Wandlergehäuse (10) aus biokompatiblem Werkstoff besteht.

6. Elektromechanischer Wandler nach Anspruch 5, dadurch gekennzeichnet, daß das Wandlergehäuse (10) mindestens zum Teil aus Metall, insbesondere Titan, Niob, Tantal und/oder deren Legierungen besteht.

7. Elektromechanischer Wandler nach Anspruch 5, dadurch gekennzeichnet, daß das Wandlergehäuse (10) mindestens zum Teil aus einem körperverträglichen keramischen Werkstoff gefertigt ist.

8. Elektromechanischer Wandler nach einem der vorhergehenden Ansprüche, dadurch gekennzeichnet, daß die Gehäusemembran (11) aus biokompatiblem Metall, insbesondere Titan, Niob, Tantal und/oder deren Legierungen besteht.

9. Elektromechanischer Wandler nach einem der vorhergehenden Ansprüche, dadurch gekennzeichnet, daß das Gehäuse (10) einen kreisförmigen Querschnitt mit einem Durchmesser im Bereichvon 6 bis 13 mm hat.

10. Elektromechanischer Wandler nach einem der vorhergehenden Ansprüche, dadurch gekennzeichnet, daß die vorzugsweise kreisförmig ausgebildete piezoelektrische Keramikscheibe (12) auf der Membraninnenseite als elektromechanisch aktives Element aufgebracht ist.

11. Elektromechanischer Wandler nach einem der vorhergehenden Ansprüche, dadurch gekennzeichnet, daß die piezoelektrische Keramikscheibe (12) aus Blei-Zirkonat-Titanat, PZT, besteht.

12. Elektromechanischer Wandler nach einem der vorhergehenden Ansprüche, dadurch gekennzeichnet, daß die Dicke der Gehäusemembran (11) und die Dicke der piezoelektrischen Keramikscheibe (12) annähernd gleich groß sind und im Bereich von 0,05 mm bis 0,15 mm liegen.

13. Elektromechanischer Wandler nach einem der vorhergehenden Ansprüche, dadurch gekennzeichnet, daß der Radius der Gehäusemembran (11) um den Faktor 1,2 bis 2,0, vorzugsweise einen Faktor von etwa 1,4, größer ist als der Radius der piezoelektrischen Keramikscheibe (12).

14. Elektromechanischer Wandler nach einem der vorhergehenden Ansprüche, dadurch gekennzeichnet, daß sowohl die Gehäusemembran (11) als auch das Gehäuse (10) elektrisch leitend sind, daß die piezoelektrische Keramikscheibe (12) mit der Gehäusemembran (11) durch eine elektrisch leitfähige Verklebung elektrisch leitend verbunden ist, und daß das Gehäuse (10) den einen (13) von zwei elektrischen Wandleranschlüssen (13, 15) bildet.

15. Elektromechanischer Wandler nach einem der vorhergehenden Ansprüche, dadurch gekennzeichnet, daß der Bügel (18) zur Übertragung der Membranschwingungen auf das Koppelelement (28, 29, 39, 40, 42, 43) zum Mittel- oder Innenohr im wesentlichen L-förmig ausgebildet und mit seinem kurzen Schenkel (19) im Bereich der Membranmitte fixiert ist, sowie daß der lange Schenkel (20) dieses Bügels im Abstand von und annähernd parallel zur Membranebene verläuft und das Ende des langen Schenkels über die Außenkante des Gehäuses (10) um eine kurze Strecke, vorzugsweise etwa 2 mm, hinausragt.

16. Elektromechanischer Wandler nach einem der vorhergehenden Ansprüche, dadurch gekennzeichnet, daß der Bügel (18) zwecks Erzielung hoher Biegesteifigkeit aus einem Werkstoff mit hohem Elastizitätsmodul aus metallischem Voll- oder Hohldraht oder kohlefaserverstärktem Verbundwerkstoff hergestellt ist, und einen Durchmesser im Bereich von 0,25 bis 1,00 mm hat.

17. Elektromechanischer Wandler nach einem der vorhergehenden Ansprüche, dadurch gekennzeichnet, daß das Koppelelement (28, 29, 39, 40, 42, 43) zum Weiterleiten der über den Bügel (18) übertragenen mechanischen Membranschwingungen auf eines der Mittelohr-Ossikel oder auf das Innenohr über ein Fenster ausgebildet ist, das aus der das ovale Fenster, das runde Fenster und ein artifizielles Fenster umfassenden Gruppe ausgewählt ist.

18. Elektromechanischer Wandler nach Anspruch 17, dadurch gekennzeichnet, daß an dem schwingenden Ende des Wandlerbügels (18) eine Stapesprothese (28) zur direkten Innenohranregung mechanisch fest fixiert ist.

19. Elektromechanischer Wandler nach Anspruch 17, dadurch gekennzeichnet, daß an dem schwingenden Ende des Wandlerbügels (18) über ein aus Platin, Platinlegierungen, Titan, Titanlegierungen, Niob, Nioblegierungen oder Edelstahl bestehendes Drahthäkchen (42) eine Hülse (39) mechanisch fest fixiert ist, die bei fehlendem oder entferntem Amboß auf das Köpfchen des Steigbügels zur mechanisch direkten Anregung des Steigbügels aufsetzbar ist und die aus Polyethylen, Polytetrafluorethylen oder Hydroxylapathit gefertigt ist.

20. Elektromechanischer Wandler nach Anspruch 17, dadurch gekennzeichnet, daß an dem schwingenden Ende des Wandlerbügels (18) der eine Hakenteil (46) eines aus Platin, Platinlegierungen, Titan, Titanlegierungen, Niob, Nioblegierungen oder Edelstahl gefertigten Doppelhakens (43) mechanisch fest fixiert ist, dessen anderer Hakenteil (44) bei intaktem Amboß-Steigbügelgelenk nach Art einer Stapesprothese um den langen Amboßfortsatz herumschlingbar ist, um die Schwingungen des Wandlerbügels auf den Amboß zu übertragen.

21. Elektromechanischer Wandler nach einem der vorhergehenden Ansprüche, dadurch gekennzeichnet, daß durch Wahl der mechanischen Eigenschaften von Wandlermembran (11), Bügel (18) und Koppelelement (28, 29, 39, 40, 42, 43) das diese Bauteile umfassende schwingfähige System so abgestimmt ist, daß seine erste mechanische Resonanzfrequenz am oberen Ende des audiologischen Übertragungsbereiches im Bereich von 6 bis 12 kHz, insbesondere bei etwa 10 kHz, liegt.

22. Elektromechanischer Wandler nach einem der vorhergehenden Ansprüche, dadurch gekennzeichnet, daß die mechanischen Eigenschaften der Wandlermembran (11) so gewählt sind, daß die mechanische Ausgangsimpedanz des Wandlers deutlich über der mechanischen Lestimpedanz bei Ankopplung der Wandlerschwingungen an das Mittel- oder das Innenohr liegt und die Auslenkung des Koppelelements (28, 29, 39, 40, 42, 43) lastunabhängig eingeprägt ist.

23. Elektromechanischer Wandler nach einem der vorhergehenden Ansprüche, dadurch gekennzeichnet, daß zur Eindämmung des Anteils der von der schwingenden Membran (11) abgestrahlten Schallenergie auf die Oberseite des Wandlergehäuses (10) ein Deckel (33) verdrehsicher aufgesetzt ist, der als Austrittsöffnung für den Bügel einen U-förmigen Schlitz (34) aufweist.

24. Elektromechanischer Wandler nach Anspruch 23, dadurch gekennzeichnet, daß der Deckel (33) auf seiner Oberseite mit mindestens einer federnden Klammer (37) versehen ist, die nach Positionierung des Wandlergehäuses (10) für dessen feste und langzeitstabile Fixierung gegen eine darüberliegende Decke einer artifiziellen Mastoidhöhle aufspreizbar ist.

25. Elektromechanischer Wandler nach Anspruch 24, dadurch gekennzeichnet, daß die Klammer (37) drahtförmig ausgebildet und aus einem Werkstoff gefertigt ist, der aus der Platin, Platinlegierungen, Titan, Titanlegierungen, Niob, Nioblegierungen und Edelstahl umfassenden Werkstoffgruppe ausgewählt ist.

26. Elektromechanischer Wandler nach Anspruch 4, dadurch gekennzeichnet, daß die Unterseite und die Stufe des Wandlergehäuses (10) mit einer bioaktiven Schicht beschichtet sind, die nach Positionierung des Wandlers mit dem Knochen des Bodens der artifiziellen Mastoidhöhle verwächst und so eine langzeitstabile, mechanisch feste Fixierung des Wandlers gewährleistet.

27. Elektromechanischer Wandler nach einem der vorhergehenden Ansprüche, dadurch gekennzeichnet, daß das Wandlergehäuse (10) nach Positionierung mit einem biokompatiblen, verklebenden Werkstoff, insbesondere Knochenzement, langzeitstabil mechanisch fest fixiert wird.

28. Elektromechanischer Wandler nach einem der Ansprüche 1 bis 26, gekennzeichnet durch eine Haltevorrichtung (48), die aus einem ein langzeitstabiles, mechanisch festes Verwachsen im Antrum gewährleistenden bioaktiven Material besteht und in welcher das Wandlergehäuse (10) mechanisch fest und auswechselbar fixierbar ist.

## Claims

1. Electromechanical transducer for implantable hearing aids for direct mechanical stimulation of the middle ear or the inner ear, characterised by a hermetically sealed and biocompatible housing (10), wherein one wall of the housing is designed as a membrane (11) which is capable of oscillation and which together with a piezoelectric ceramic disc (12) placed on the inside constitutes an electromechanically active heteromorphous composite element and the mechanical oscillations of which are transmitted via a mechanically rigid transmitting element (18) fixed securely on the outside of the membrane together with a mechanically rigid coupling element (28, 29, 39, 40, 42, 43) to an ossicle of the middle ear or directly to the inner ear.

2. Electromechanical transducer according to Claim 1, characterised in that the mechanically rigid transmitting element (18) is fixed at least approximately to the midpoint of the membrane.

3. Electromechanical transducer according to Claim 1 or 2, characterised in that the upper side of the transducer housing (10) is the membrane (11) and the membrane (11) is circular.

4. Electromechanical transducer according to any one of the preceding claims, characterised in that the underside of the transducer housing (10) is constructed to be stepped in such a way that the transducer housing can be positioned in an artificially created mastoid cavity adjacent to the tympanic cavity in the antrum in such a way that the membrane region is situated close to the ossicles of the middle ear, the step of the underside of the housing is mounted on the osseous floor of the mastoid cavity and it is possible to couple the oscillating transducer component to the middle ear or to the inner ear in the clear view of the operator.

5. Electromechanical transducer according to any one of the preceding claims, characterised in that the transducer housing (10) consists of biocompatible material.

6. Electromechanical transducer according to Claim 5, characterised in that the transducer housing (10) consists at least partly of metal, in particular titanium, niobium, tantalum and/or alloys thereof.

7. Electromechanical transducer according to Claim 5, characterised in that the transducer housing (10) is manufactured at least partly from a body-compatible ceramic material.

8. Electromechanical transducer according to any one of the preceding claims, characterised in that the housing membrane (11) consists of biocompatible metal, in particular titanium, niobium, tantalum and/or alloys thereof.

9. Electromechanical transducer according to any one of the preceding claims, characterised in that the housing (10) has a circular cross-section with a diameter in the range from 6 to 13 mm.

10. Electromechanical transducer according to any one of the preceding claims, characterised in that the piezoelectric ceramic disc (12), which is preferably constructed to be circular, is placed on the inside of the membrane by way of electromechanically active element.

11. Electromechanical transducer according to any one of the preceding claims, characterised in that the piezoelectric ceramic disc (12) consists of lead zirconate titanate, PZT.

12. Electromechanical transducer according to any one of the preceding claims, characterised in that the thickness of the housing membrane (11) and the thickness of the piezoelectric ceramic disc (12) are of approximately equal magnitude and lie in the range from 0.05 mm to 0.15 mm.

13. Electromechanical transducer according to any one of the preceding claims, characterised in that the radius of the housing membrane (11) is greater then the radius of the piezoelectric ceramic disc (12) by a factor of 1.2 to 2.0, preferably by a factor of about 1.4.

14. Electromechanical transducer according to any one of the preceding claims, characterised in that both the housing membrane (11) and the housing (10) are electrically conductive, the piezoelectric ceramic disc (12) is connected in electrically conductive manner to the housing membrane (11) by means of an electrically conductive adhesive bond, and the housing (10) constitutes one (13) of two electrical transducer connections (13, 15).

15. Electromechanical transducer according to any one of the preceding claims, characterised in that the transmitting element (18) for transmitting the membrane oscillations to the coupling element (28, 29, 39, 40, 42, 43) leading to the middle ear or the inner ear is designed so as to be substantially L-shaped and is fixed by its short arm (19) in the region of the midpoint of the membrane, and the long arm (20) of this transmitting element extends at a distance from and approximately parallel to the plane of the membrane and the end of the long arm projects beyond the outer edge of the housing (10) by a short distance, preferably by about 2 mm.

16. Electromechanical transducer according to any one of the preceding claims, characterised in that for the purpose of achieving high bending resistance the transmitting element (18) is manufactured from a material having a high modulus of elasticity, from a metallic solid wire or hollow wire or carbon-fibre-reinforced composite material, and has a diameter in the range from 0.25 to 1.00 mm.

17. Electromechanical transducer according to any one of the preceding claims, characterised in that the coupling element (28, 29, 39, 40, 42, 43) is designed for passing on the mechanical membrane oscillations transmitted via the transmitting element (18) to one of the ossicles of the middle ear or to the inner ear via a window which is selected from the group comprising the oval window, the round window and an artificial window.

18. Electromechanical transducer according to Claim 17, characterised in that a stapes prosthesis (28) is fixed in mechanically secure manner to the oscillating end of the transducer transmitting element (18) with a view to direct stimulation of the inner ear.

19. Electromechanical transducer according to Claim 17, characterised in that a sheath (39) is fixed in mechanically secure manner to the oscillating end of the transducer transmitting element (18) via a wire hook (42) consisting of platinum, platinum alloys, titanium, titanium alloys, niobium, niobium alloys or high-grade steel, whereby in the case of an absent or removed incus said sheath is capable of being placed on the stapes head with a view to mechanically direct stimulation of the stapes and is manufactured from polyethylene, polytetrafluoroethylene or hydroxylapatite.

20. Electromechanical transducer according to Claim 17, characterised in that one hooked section (46) of a double hook (43) manufactured from platinum, platinum alloys, titanium, titanium alloys, niobium, niobium alloys or high-grade steel is fixed in mechanically secure manner to the oscillating end of the transducer transmitting element (18), the other hooked section (44) of said double hook being capable, in the case of an intact incus-stapes joint, of being placed about the long incus extension in the manner of a stapes prosthesis in order to transmit the oscillations of the transducer transmitting element to the incus.

21. Electromechanical transducer according to any one of the preceding claims, characterised in that by the choice of the mechanical properties of transducer membrane (11), transmitting element (18) and coupling element (28, 29, 39, 40, 42, 43) the system which is capable of oscillation and which comprises these components is tuned in such a way that its first mechanical resonance frequency lies at the upper end of the audiological transmission range in the range from 6 to 12 kHz, in particular at around 10 kHz.

22. Electromechanical transducer according to any one of the preceding claims, characterised in that the mechanical properties of the transducer membrane (11) are chosen in such a way that the mechanical output impedance of the transducer lies clearly above the mechanical load impedance in the case of coupling of the transducer oscillations to the middle ear or to the inner ear and the deflection of the coupling element (28, 29, 39, 40, 42, 43) is impressed in a manner which is not dependent on the load.

23. Electromechanical transducer according to any one of the preceding claims, characterised in that with a view to stemming the proportion of acoustical energy radiated from the oscillating membrane (11) a cover (33) is mounted in torsion-resistant manner onto the upper side of the transducer housing (10), said cover exhibiting a U-shaped slot (34) by way of outlet for the transmitting element.

24. Electromechanical transducer according to Claim 23, characterised in that the cover (33) is provided on its upper side with at least one resilient clip (37) which after positioning of the transducer housing (10) for the secure and long-term stable fixing thereof is capable of being opened out towards a covering of an artificial mastoid cavity situated above it.

25. Electromechanical transducer according to Claim 24, characterised in that the clip (37) is designed to take the form of a wire and is manufactured from a material selected from the group of materials comprising platinum, platinum alloys, titanium, titanium alloys, niobium, niobium alloys and high-grade steel.

26. Electromechanical transducer according to Claim 4, characterised in that the underside and the step of the transducer housing (10) are coated with a bioactive layer which after positioning of the transducer grows together with the bone of the floor of the artificial mastoid cavity and so ensures a mechanically secure, long-term stable fixing of the transducer.

27. Electromechanical transducer according to any one of the preceding claims, characterised in that after positioning with a biocompatible sealing material, in particular bone cement, the transducer housing (10) is fixed so as to be mechanically secure and stable in the long term.

28. Electromechanical transducer according to one of Claims 1 to 26, characterised by a retaining device (48) which consists of a bioactive material ensuring a growing-together in the antrum that is mechanically secure and stable in the long term and in which the transducer housing (10) is capable of being fixed so as to be mechanically secure and replaceable.

## Revendications

1. Convertisseur électromécanique pour appareils de correction auditive implantables, destiné à effectuer une excitation mécanique directe de l'oreille moyenne ou interne, caractérisé par un boîtier (10) hermétiquement clos et biocompatible dont une paroi est conçue sous la forme d'une membrane (11) capable d'osciller qui, avec une plaque céramique piézo-électrique (12) appliquée sur le côté intérieur, constitue un élément composite hétéromorphe à action électromécanique et dont les oscillations mécaniques sont transmises par une pièce courbe (18) mécaniquement rigide, solidarisé au côté extérieur de la membrane, conjointement avec un élément d'accouplement mécaniquement rigide (28, 29, 39, 40, 42, 43), à un osselet de l'oreille moyenne ou directement à l'oreille interne.

2. Convertisseur électromécanique selon la revendication 1, caractérisé en ce que la pièce courbe mécaniquement rigide (18) est fixée au moins sensiblement au centre de la membrane.

3. Convertisseur électromécanique selon la revendication 1 ou 2, caractérisé en ce que le dessus du boîtier (10) du convertisseur est constitué par la membrane (11) et en ce que la membrane (11) est de forme circulaire.

4. Convertisseur électromécanique selon l'une des revendications précédentes, caractérisé en ce que le dessous du boîtier (10) du convertisseur est conçu de manière étagée, de manière que le boîtier du convertisseur puisse être positionné dans une cavité mastoïdienne créée artificiellement, de façon adjacente à la caisse du tympan dans l'antre, de manière que la zone de la membrane se trouve à proximité des osselets de l'oreille moyenne, que l'étage du dessous du boîtier repose sur le fond osseux de la cavité mastoïdienne et que la partie oscillante du convertisseur puisse être reliée à l'oreille moyenne ou à l'oreille interne sans gêner la visibilité de l'opérateur.

5. Convertisseur électromécanique selon l'une des revendications précédentes, caractérisé en ce que le boîtier (10) du convertisseur est réalisé en matériau biocompatible.

6. Convertisseur électromécanique selon la revendication 5, caractérisé en ce que le boîtier (10) du convertisseur est réalisé au moins partiellement en métal, en particulier en titane, en niobium, en tantale et/ou en leurs alliages.

7. Convertisseur électromécanique selon la revendication 5, caractérisé en ce que le boîtier (10) du convertisseur est fabriqué, au moins partiellement, dans un matériau céramique biocompatible.

8. Convertisseur électromécanique selon l'une des revendications précédentes, caractérisé en ce que la membrane (11) du boîtier est réalisée dans un métal biocompatible, en particulier en titane, en niobium, en tantale et/ou en leurs alliages.

9. Convertisseur électromécanique selon l'une des revendications précédentes, caractérisé en ce que le boîtier (10) a une section transversale circulaire avec un diamètre compris entre 6 et 13 mm.

10. Convertisseur électromécanique selon l'une des revendications précédentes, caractérisé en ce que la plaque céramique piézo-électrique de préférence circulaire (12) est disposée sur le côté intérieur de la membrane sous la forme d'un élément à action électromécanique.

11. Convertisseur électromécanique selon l'une des revendications précédentes, caractérisé en ce que la plaque céramique piézo-électrique (12) est réalisée en plomb-zirconate-titanate, PZT.

12. Convertisseur électromécanique selon l'une des revendications précédentes, caractérisé en ce que l'épaisseur de la membrane (11) du boîtier et l'épaisseur de la plaque céramique piézo-électrique (12) sont sensiblement identiques et sont comprises entre 0,05 mm et 0,15 mm.

13. Convertisseur électromécanique selon l'une des revendications précédentes, caractérisé en ce que le rayon de la membrane (11) du boîtier est supérieur, d'un facteur de 1,2 à 2,0, de préférence d'un facteur d'environ 1,4, au rayon de la plaque céramique piézo-électrique (12).

14. Convertisseur électromécanique selon l'une des revendications précédentes, caractérisé en ce qu'aussi bien la membrane (11) du boîtier que le boîtier (10) sont électroconducteurs, en ce que la plaque céramique piézo-électrique (12) est reliée de manière électroconductrice à la membrane (11) du boîtier par un collage électroconducteur, et en ce que le boîtier (10) forme l'une (13) des deux connexions électriques (13, 15) du convertisseur.

15. Convertisseur électromécanique selon l'une des revendications précédentes, caractérisé en ce que la pièce courbe (18) destinée à transmettre les oscillations de la membrane à l'élément d'accouplement (28, 29, 39, 40, 42, 43) avec l'oreille moyenne ou avec l'oreille interne présente sensiblement la forme d'un L et est fixée, par sa branche courte (19), dans la zone du centre de la membrane, et en ce que la branche longue (20) de cette pièce courbe s'étend à distance du, et de manière sensiblement parallèle au, plan de la membrane et l'extrémité de la branche longue dépasse du bord extérieur du boîtier (10) sur une courte distance, de préférence sur environ 2 mm.

16. Convertisseur électromécanique selon l'une des revendications précédentes, caractérisé en ce que, pour obtenir une rigidité à la flexion élevée, la pièce courbe (18) est fabriquée dans un matériau à module d'élasticité élevé, en fil métallique plein ou creux, ou dans un matériau composite renforcé aux fibres de carbone, et possède un diamètre compris entre 0,25 et 1,00 mm.

17. Convertisseur électromécanique selon l'une des revendications précédentes, caractérisé en ce que, pour propager à l'un des osselets de l'oreille moyenne ou à l'oreille interne les oscillations mécaniques de la membrane transmises par la pièce courbe (18), l'élément d'accouplement (28, 29, 39, 40, 42, 43) est réalisé à travers une fenêtre qui est choisie parmi un groupe comprenant la fenêtre ovale, la fenêtre ronde et une fenêtre artificielle.

18. Convertisseur électromécanique selon la revendication 17, caractérisé en ce qu'une prothèse d'étrier (28) est solidarisée mécaniquement à l'extrémité oscillante de la pièce courbe (18) du convertisseur pour exciter directement l'oreille interne.

19. Convertisseur électromécanique selon la revendication 17, caractérisé en ce qu'à l'extrémité oscillante de la pièce courbe (18) du convertisseur est solidarisé mécaniquement, par l'intermédiaire d'un crochet (42) en fil de platine, d'alliages de platine, de titane, d'alliages de titane, de niobium, d'alliages de niobium ou d'acier fin, un manchon (39) qui, lorsque l'enclume est absente ou supprimée, peut reposer sur la tête de l'étrier pour effectuer une excitation mécanique directe de celui-ci et qui est réalisé en polyéthylène, en polytétrafluoréthylène ou en hydroxylapatite.

20. Convertisseur électromécanique selon la revendication 17, caractérisé en ce que l'une des parties (46) d'un crochet double (43) en platine, en alliages de platine, en titane, en alliages de titane, en niobium, en alliages de niobium ou en acier fin est solidarisée mécaniquement à l'extrémité oscillante de la pièce courbe (18) du convertisseur, l'autre partie (44) du crochet pouvant être entourée, lorsque l'articulation incudo-stapédienne est intacte, autour du prolongement long de l'enclume à la manière d'une prothèse d'étrier afin de transmettre les oscillations de l'étrier du convertisseur à l'enclume.

21. Convertisseur électromécanique selon l'une des revendications précédentes, caractérisé en ce qu'en choisissant les caractéristiques mécaniques de la membrane (11) du convertisseur, de la pièce courbe (18) et de l'élément d'accouplement (28, 29, 39, 40, 42, 43), le système oscillant composé de ces éléments est accordé pour que sa première fréquence de résonance mécanique se trouve à l'extrémité supérieure de la plage de transmission audiologique comprise entre 6 et 12 kHz, en particulier à environ 10 kHz.

22. Convertisseur électromécanique selon l'une des revendications précédentes, caractérisé en ce que les caractéristiques mécaniques de la membrane (11) du convertisseur sont choisies pour que l'impédance mécanique de sortie du convertisseur soit nettement supérieure à l'impédance mécanique de charge lors de la transmission des oscillations du convertisseur à l'oreille moyenne ou à l'oreille interne et pour que la déviation de l'élément d'accouplement (28, 29, 39, 40, 42, 43) soit appliquée de manière indépendante de la charge.

23. Convertisseur électromécanique selon l'une des revendications précédentes, caractérisé en ce que, pour arrêter la proportion d'énergie acoustique émise par la membrane oscillante (11), un couvercle (33) est posé, sans possibilité de rotation, sur le dessus du boîtier (10) du convertisseur et comporte une fente en U (34) faisant fonction d'ouverture de sortie pour la pièce courbe.

24. Convertisseur électromécanique selon la revendication 23, caractérisé en ce que le couvercle (33) est muni, sur le dessus, d'au moins une attache élastique (37) qui, après la mise en place du boîtier (10) du convertisseur, peut se déployer contre le plafond, situé au-dessus, d'une cavité mastoïdienne artificielle pour immobiliser le boîtier (10) du convertisseur de manière solide et stable dans le temps.

25. Convertisseur électromécanique selon la revendication 24, caractérisé en ce que l'attache (37) est en forme de fil métallique et est réalisée dans un matériau qui est choisi parmi le groupe de matériaux comprenant le platine, les alliages de platine, le titane, les alliages de titane, le niobium, les alliages de niobium et l'acier fin.

26. Convertisseur électromécanique selon la revendication 4, caractérisé en ce que le dessous et l'étage du boîtier (10) du convertisseur sont revêtus d'une couche bioactive qui, après la mise en place du convertisseur, adhère à l'os du fond de la cavité mastoïdienne artificielle et garantit ainsi une immobilisation stable dans le temps et mécaniquement solide du convertisseur.

27. Convertisseur électromécanique selon l'une des revendications précédentes, caractérisé en ce qu'après avoir été mis en place avec un matériau adhésif biocompatible, en particulier avec un ciment pour os, le boîtier (10) du convertisseur est immobilisé mécaniquement de manière stable dans le temps.

28. Convertisseur électromécanique selon l'une des revendications 1 à 26, caractérisé par un dispositif de retenue (48) qui est réalisé dans un matériau bioactif garantissant, dans l'antre, une adhérence mécaniquement solide et stable dans le temps et dans lequel le boîtier (10) du convertisseur peut être immobilisé de manière mécaniquement solide et avec possibilité de remplacement.
